# EUROPEAN PATENT APPLICATION

(11) **EP 3 888 742 A1**
(43) Date of publication of application: **06.10.2021**
(21) Application number: 21165293.8
(22) Date of filing: 26.03.2021
(51) Int. Cl.: A61N 2/02

(54) **A THERAPEUTIC DEVICE**

(30) Priority: 01.04.2020 IT 202000006889
(71) Applicant: Cehic, Aldo, 37135 Verona (IT)
(72) Inventor: Cehic, Aldo, 37135 Verona (IT)
(74) Representative: Trentin, Michele

(57) **Abstract**

A therapeutic device for infusing bioenergy on a limited area of the skin surface of the human body comprising: a gripping element (2) to a body segment surrounding the limited area of interest; an element made of FIR fabric (7) stably coupled to the gripping element (2) to be put in contact with the limited area of interest; an oscillating circuit (10) stably coupled to the FIR fabric element (7) to generate a magnetic field capable of hitting the limited area of interest.

## Description

### Definitions

In the present document, the acronym FIR refers to far infrared ray.

FIR fabric is intended as a fabric capable of absorbing and emitting far infrared rays.

### Field of application

The present invention is applicable in the field of healthcare and, in particular, relates to devices for therapeutic-healthcare treatment.

More in detail, the present invention relates to devices for the physiotherapeutic treatment of the human body and for the achievement of a state of well-being in general.

### Background art

In the field of human body care, alternative or complementary methods of care to classical medicine are becoming increasingly common.

Many of such methods are attributable to the fields of analgesia or symptomatic therapy, i.e., to fields which deal with pain management therapies, contractive muscle diseases or other.

In all such situations, classical medicine proposes the use of drugs, be they NSAIDs (non-steroidal anti-inflammatory drugs) or non-NSAIDs (opiates, cortisones) aimed at anaesthetizing more or less large parts of the human body or at causing the relaxation thereof. However, it is also known that the use of drugs regardless involves contraindications such that it is good practice not to abuse them.

In this sense, a plurality of alternative care methods have emerged, some particularly common examples of which are acupuncture, chromotherapy and some forms of physical massage.

Generally they are based on or derive from oriental practices and refer thereto not only for the method used, but also for the identification of the points to be treated on the body and for the objectives to be achieved through action on such points.

In this sense, for example, according to Chinese medicine, acting on specific points on the surface of the human body has a direct effect on corresponding vital organs and functions.

In particular, so-called "meridians" are identified on the body which are non other than channels of bioenergy which affect various organs and various body functions. By acting on the points of the body surface where such meridians flow, it is possible to indirectly affect such organs and such functions.

Moreover, a particular point of the human body is the palm of the wrist, since it embodies twelve of such points which are in direct so-called "energetic" contact with twelve internal organs.

It is also known from numerous studies that the human body, at least through body heat, emits infrared radiation in the wavelength spectrum of far infrared rays or FIRs. Such rays, which also correspond to rays emitted by the sun, in fact correspond to a loss or dispersion of bioenergy of the human body. In other words, from a point of view of physics, this biothermic phenomenon is configured as dispersed bioenergy which is not used for the various body functions.

### Presentation of the invention

The object of the present invention is to use the above-mentioned knowledge to act on the main organs of the human body and on the main body functions in order to improve the psychic and physical-energetic state of the person.

Within the scope of this general object, a particular object of the present invention is to provide a therapeutic device which allows to recover at least a part of the body bioenergy unnecessarily dispersed by the human body.

Another object is to provide a therapeutic device which allows such bioenergy to be redirected to one or more of the organs or functions of the human body to improve the functioning thereof and stimulate a state of well-being.

A further object of the present invention is to provide a therapeutic device which allows to act selectively on such organs and in general to act selectively on the human body.

Said objects, as well as others which will become clearer below, are achieved by a therapeutic device according to the following claims, which are to be considered an integral part of the present patent.

In particular, the therapeutic device allows the infusion of photonic and biomagnetic bioenergy on a limited area of the skin surface of the human body and comprises firstly at least one element for gripping at least one body segment surrounding the limited area of interest for the treatment.

There is also at least one element made of FIR fabric stably coupled to the gripping element to be brought into contact with the limited area just highlighted.

According to an aspect of the invention, the device also comprises at least one oscillating circuit without electric power supply permanently coupled to the FIR fabric element to obtain a magnetic field capable of hitting the limited area of interest of the treatment.

Advantageously, the FIR fabric allows to reflect the far infrared rays emitted by the human body towards the same area of interest as the skin surface which emitted them so as to recover them.

Still advantageously, the oscillating circuit generates a magnetic field since it is supplied by the rays emitted by the human body. Such a magnetic field also helps to re-fuse bioenergy on the user's skin surface while focusing the far infrared rays reflected by the FIR fabric.

Advantageously, therefore, not only are FIR rays returned to the human body, but they are also focused on a restricted area by virtue of the influence of the oscillating circuit.

Still advantageously, the gripping element allows to arrange the therapeutic device on selected areas of interest and typically corresponding to meridian points or in any case to points highlighted by oriental medicine.

In particular, in accordance with an aspect of the invention, the gripping element is a bracelet. This allows to arranged the FIR fabric element and the oscillating circuit at the palms of the wrists, i.e., the twelve points highlighted by Chinese medicine. It follows, still advantageously, that the therapeutic device of the invention in this case acts simultaneously on as many different organs and functions of the human body.

### Brief description of the drawings

Further features and advantages of the invention will become more evident in light of the detailed description of a preferred but not exclusive embodiment of a therapeutic device according to the invention, illustrated by way of non-limiting example with the aid of the accompanying drawings, in which:

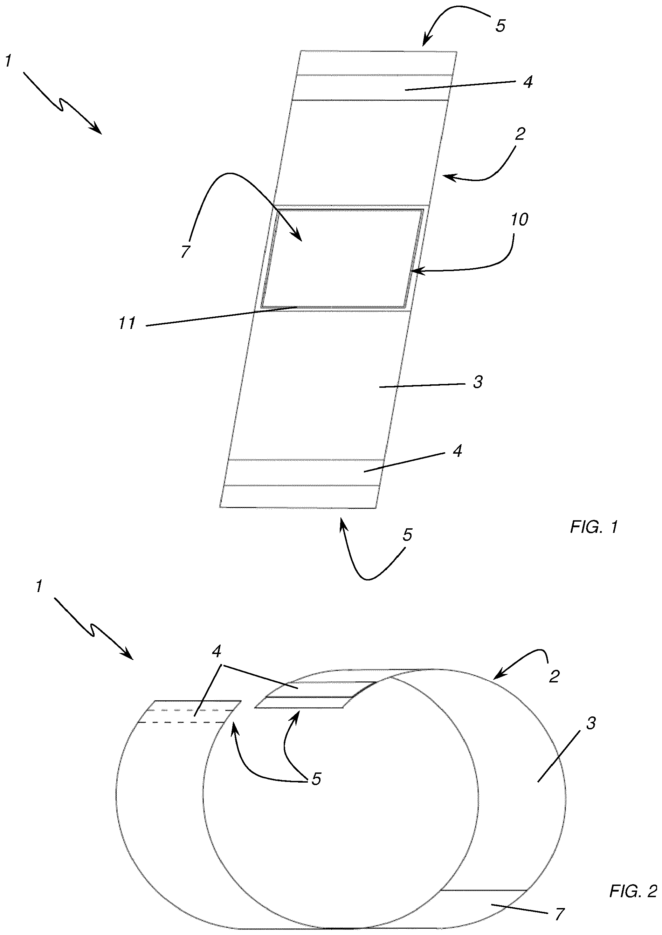

- FIG. 1 depicts a therapeutic device according to the invention in an axonometric view;
- FIG. 2 depicts the therapeutic device of FIG 1 in an operating mode arrangement step.

### Detailed description of an exemplary preferred embodiment

With reference to the aforementioned figures, a therapeutic device **1** shaped to infuse bioenergy on a limited area of a user's skin surface is described herein.

In order to operate in the aforementioned sense, the therapeutic device **1** first comprises an element for gripping **2** to a body segment surrounding the limited area of interest of the user's body.

In the figures it can be observed that the gripping element **2** is formed by a bracelet **3.** This makes the therapeutic device **1** easily applicable to the terminal parts of the human body such as wrists or ankles.

Obviously, such an aspect must not be considered limiting for different embodiments of the invention where the gripping element is formed, for example, by bands of various length and size (also applicable to wrists or ankles), patches or other. All such embodiments allow the therapeutic device **1** of the invention to be applied on any limited area of the skin surface of the human body.

In order to guarantee the position of the bracelet **3,** according to the embodiment described, it comprises Velcro® **4** coupled to the ends **5** of the bracelet itself.

Obviously such an aspect must also not be considered limiting for different embodiments of the invention where different types of closures are used such as male/female metal elements, buckles, button-hole systems, or other.

According to another aspect of the invention, an element made of FIR fabric **7** is stably coupled to the gripping element **2** so as to be brought into contact with the limited area of interest of the user's skin surface.

Advantageously, therefore, the therapeutic device **1** of the invention, at the aforementioned limited area, creates a barrier for the FIR rays emitted by the human body, reflecting them on the same area.

Still advantageously, such FIR rays are reinfused into the user's body, returning bioenergy which would be unnecessarily dispersed. Moreover, since the therapeutic device **1** is shaped to be arranged at areas which according to Chinese medicine energetically refer to internal organs or body functions of interest, advantageously such reinfusion allows to increase the amount of bioenergy and therefore the well-being of such organs and of the organism as a whole.

It is evident that the number of elements made of FIR fabric is not a limiting feature for the invention, being able to be in any quantity so as to cover different areas which may be separate from each other or, if overlapping, so as to increase the effectiveness of the reflecting barrier.

In this sense, however, according to another aspect of the invention, the gripping element **2** is also at least partially made of FIR fabric. Thereby, advantageously, the reflective effect of the barrier is increased at the limited area of the skin surface affected by the therapeutic device **1** of the invention without increasing the thicknesses thereof.

According to another aspect of the invention, the therapeutic device **1** also comprises an oscillating circuit **10** without electric power supply and permanently coupled to the FIR fabric element **7** to create a magnetic field capable of hitting the limited area of interest.

Still advantageously, the oscillating circuit **10** generates a magnetic field since it is supplied by the rays emitted by the human body. Such a magnetic field also helps to re-fuse bioenergy on the user's skin surface while focusing the far infrared rays reflected by the FIR fabric element **7.**

Advantageously, therefore, not only are the FIR rays returned to the human body, but they are also focused on a restricted area by virtue of the influence of the oscillating circuit **10.**

Going into the detail of the embodiment of the invention which is described, the oscillating circuit comprises a shaped closed coil **11** made of electrically conductive and advantageously insulated material to avoid contacts with the human body. Obviously, such an aspect must also not be considered limiting for the invention, the number of turns being any so as to obtain a coil.

Typically, but not necessarily, the oscillating circuit **10** has an oscillation frequency in the infrared frequency band, but such an aspect must also not be considered limiting for different embodiments of the invention.

With regard to the manufacture of the FIR fabric element **7** and the gripping element **2,** according to the embodiment described, they are both made of elastic fabric. Obviously, such an aspect must also not be considered limiting for the invention.

Furthermore, with reference to the gripping element **2,** it can be made in different colours to add to the normal treatment included for the therapeutic device **1** of the invention, also a chromotherapeutic function. Moreover, in such a case, it is possible to include to act also according to the Caldaica sequence in which each day of the week has its own colour.

Operatively, the user arranges the therapeutic device at one or more points of those indicated in Chinese medicine or other forms of medicine. For example, the user can proceed to arrange the therapeutic device **1** such that the FIR fabric element **7** and the oscillating circuit **10** are at the palm of the wrist. More specifically, wanting to interest the points of Chinese medicine discussed above, the FIR fabric element **7** and the oscillating circuit **10** are positioned at the anatomical arrangement corresponding to the classic wrist grip of traditional Chinese medicine.

In any case, from the outset the FIR rays emitted by the body excite the oscillating circuit **10** which consequently generates a magnetic field incident on the same limited area of interest. In addition, the FIR fabric element **7** prevents the emitted FIR rays from dispersing into the environment by reflecting them towards the same limited area. Moreover, the latter are also focused in such a direction by the magnetic field generated by the oscillating circuit **10,** maximizing the effects thereof.

The aforementioned treatment can be carried out continuously or at precise times of the day at which the organs affected by the treatment are more receptive in accordance with chronobiology.

Having said the above, it is clear that the therapeutic device of the invention achieves all the intended objectives.

In particular, if correctly applied, it acts at the reflexogenic areas related to the main organs of the human body and on the main body functions in order to improve the psychic and physical-bioenergetic state of the person.

In particular, the therapeutic device of the invention allows to recover at least a part of the infrared type body bioenergy unnecessarily dispersed by the human body by redirecting it towards the user's skin surface. Consequently, if the therapeutic device is properly applied, such bioenergy is redirected to one or more of the organs or functions of the human body to improve the functioning thereof and restore an increased state of well-being and greater physical bioenergy to the person.

More specifically, the therapeutic device of the invention also allows to selectively act on such organs and in general to selectively act on the human body.

The invention might be subject to many changes and variants, which are all included in the appended claims. Moreover, all the details may furthermore be replaced by other technically equivalent elements, and the materials may be different depending on the needs, without departing from the protection scope of the invention defined by the appended claims.

## Claims

1. A therapeutic device for infusing bioenergy over a limited area of a skin surface of a human body, comprising:
- at least one gripping element (2) to at least one body segment surrounding the limited area of interest;
- at least one element made of FIR fabric (7) stably coupled to said gripping element (2) to be brought into contact with the limited area of interest;
- at least one oscillating circuit (10) without electric power supply and permanently coupled to said FIR fabric element (7) so as to capture at least body bioenergy rays emitted by the human body and generate a magnetic field capable of hitting the limited area of interest.

2. Therapeutic device according to claim 1, **wherein** said oscillating circuit (10) comprises a single shaped closed coil (11) made of electrically conductive material.

3. Therapeutic device according to claim 1, **wherein** said oscillating circuit comprises a coil closed thereon, made of electrically conductive material and comprising two or more turns.

4. Therapeutic device according to any one of the preceding claims, **wherein** said gripping element (2) is a bracelet (3).

5. Therapeutic device according to any one of claims 1 to 3, **wherein** said gripping element is a patch.

6. Therapeutic device according to any one of claims 1 to 3, **wherein** said gripping element is a band.

7. Therapeutic device according to any one of the preceding claims, **wherein** said gripping element (2) is at least partially made of FIR fabric.

8. Therapeutic device according to any of the preceding claims, **wherein** the oscillation frequency of said oscillating circuit (10) is in the infrared frequency band.
